# FASCICULE DE BREVET EUROPEEN

(11) **EP 4 167 850 B1**
(45) Date de publication et mention de la délivrance du brevet: **10.07.2024**
(21) Numéro de dépôt: 21732330.2
(22) Date de dépôt: 17.06.2021
(51) Int. Cl.: A61B 5/087, A61B 5/00, A61B 5/1455

(54) **MÉTHODE POUR LA GÉNÉRATION D'UNE DONNÉE RESPIRATOIRE ET DISPOSITIF ASSOCIÉ**
VERFAHREN ZUR ERZEUGUNG EINES ATEMDATUMS UND ZUGEHÖRIGE VORRICHTUNG
METHOD FOR GENERATING A RESPIRATORY DATUM AND ASSOCIATED DEVICE

(30) Priorité: 17.06.2020 FR 2006327
(43) Date de publication de la demande: 26.04.2023
(73) Titulaire: Happlyz Medical, 60350 Pierrefonds (FR)
(72) Inventeur: LESOBRE, Vanessa, 78400 CHATOU (FR); BERRIOT, Maxime, 78400 CHATOU (FR)
(74) Mandataire: Oak & Fox
(86) Numéro de dépôt international: PCT/EP2021/066408
(87) Numéro de publication internationale: WO 2021/255168

(56) Documents cités:
- WO-A1-2017/072036
- WO-A2-2013/067495
- CN-A- 105 913 721
- US-A1- 2015 027 294
- US-A1- 2019 134 460
- US-A1- 2019 209 044

## Description

### Domaine de l'invention

L'invention concerne une méthode et un dispositif associé pour la génération d'une donnée respiratoire.

### État de la technique

L'expiration est une expulsion volontaire d'air par le relâchement du diaphragme et la contraction des muscles intercostaux. La pression exercée ainsi sur les alvéoles pulmonaires libère l'air qu'elles contiennent.

Cependant, certaines maladies telles que la mucoviscidose, la bronchopneumopathie chronique obstructive, l'asthme peuvent affecter ces muscles au point de causer des insuffisances respiratoires. Il a également été observé que des patients guéris de la Covid19 pouvaient être sujets à des insuffisances respiratoires pendant plusieurs mois après leur guérison.

On connait un dispositif d'entrainement dans lequel le sujet aspire dans un tube relié à des canaux dans lesquels sont placées des boules qui vont être soulevées par l'expiration du sujet. Cependant, ce type de système est monotone est peut devenir ennuyeux pour le patient. Or, plusieurs études ont démontré une faible observance des patients pour les maladies chroniques telles que l'asthme. Un tel système présente donc un risque de mauvais suivi par le patient de son traitement, et donc de ne pas atteindre les résultats optimaux.

On connait également le document WO2018/011358 permettant aux sportifs de travailler et d'analyser leurs capacités respiratoires grâce à une pièce buccale permettant de mesurer la pression d'air expirée et d'analyser cette pression d'air. Un inconvénient de ce système est qu'il n'est pas adapté à des personnes non sportives qui se lassent vite d'exercices répétitifs et qui n'ont pas la maitrise totale de leur respiration, notamment au niveau du rythme et de la réactivité des muscles respiratoires. Le document WO2017072036 A1 divulgue un dispositif de génération d'une donnée respiratoire d'un utilisateur comprenant une unité de respiration avec un capteur de pression et une interface tactile, ainsi qu'un terminal électronique comprenant un calculateur adapté pour exécuter: une génération d'une consigne multimédia horodatée, une mesure d'une pression d'air, une génération d'un indicateur respiratoire horodaté et une détection d'une interaction sur l'interface tactile.

L'invention vise donc à fournir une méthode et un dispositif associé permettant à des utilisateurs de suivre et d'améliorer leur performance respiratoire.

### Résumé de l'invention

Selon un aspect, l'invention concerne une méthode de génération d'une donnée respiratoire d'un utilisateur.

Ladite méthode comprend :
- une génération d'une consigne multimédia horodatée par un logiciel d'un terminal électronique et la transmission de cette consigne multimédia à l'utilisateur par un moyen de transmission ;
- une mesure d'une pression d'air dans une chambre fluidique d'expiration d'une unité de respiration pour recevoir un volume d'air expiré et/ou inspiré d'un utilisateur ;
- une génération d'un indicateur respiratoire horodaté en fonction de la pression d'air mesurée,
- une détection d'une interaction sur une interface tactile, par exemple une interface tactile solidaire de l'unité de respiration ;
- une génération d'un indicateur tactile horodaté en fonction de l'interaction détectée ;
- une réception par un calculateur du terminal électronique de l'indicateur respiratoire et de l'indicateur tactile ;
- une génération d'une donnée respiratoire quantifiant une performance respiratoire de l'utilisateur en fonction de la corrélation de l'indicateur tactile et de l'indicateur respiratoire avec la consigne multimédia.

Un avantage de l'invention est de générer une donnée permettant de mesurer les progrès respiratoires d'un utilisateur. Un autre avantage est d'inciter l'utilisateur à synchroniser ses mouvements d'interaction avec son expiration et/ou son inspiration.

Dans un mode de réalisation, l'indicateur respiratoire est généré en fonction de :
- une valeur de pression d'air maximale ou moyenne mesurée sur un intervalle de temps prédéterminé, et/ou
- une durée pendant laquelle la valeur de pression d'air est au-dessus d'un seuil prédéterminé.

Dans un mode d'exécution, l'indicateur tactile comprend en outre un identifiant d'un moyen d'interaction sur lequel a été détectée une interaction ; et/ou la durée de l'interaction ; et/ou l'intensité de l'interaction. Un avantage est de discriminer chaque interface tactile (ou « touche » de l'unité). De cette manière, l'utilisateur peut obtenir un effet différent en fonction de la touche sur laquelle il appuie et/ou la consigne multimédia peut comprendre une instruction d'interaction tactile sur une touche. Dans un mode de réalisation, le dispositif comprend une manette adaptée pour être tenue dans une main de l'utilisateur et comprenant à sa surface ladite interface tactile.

Dans un mode d'exécution, la consigne multimédia horodatée comprend une instruction de respiration et/ou une instruction d'interaction.

Un avantage de la présence de consignes tactiles est de permettre à l'utilisateur de moins se focaliser sur sa respiration, et donc d'obtenir des résultats plus représentatifs des capacités de l'utilisateur. D'autre part, les consignes tactiles permettent à l'utilisateur de fixer une synchronicité sur laquelle il peut s'appuyer pour synchroniser ses expirations.

Dans un mode d'exécution, l'instruction de respiration comprend une date de départ et dans laquelle la donnée respiratoire est générée en fonction de la date de départ et de l'horodatage de l'indicateur respiratoire.

Dans un mode d'exécution, l'instruction de respiration comprend une valeur à atteindre. Dans un mode d'exécution, l'indicateur respiratoire comprend une pression maximale ou moyenne mesurée. Dans un mode d'exécution, la donnée respiratoire est générée en fonction de la ladite valeur à atteindre et de ladite pression maximale ou moyenne mesurée.

Dans un mode d'exécution, l'instruction d'interaction comprend une date de départ et dans laquelle la donnée respiratoire est générée en fonction de ladite date de départ et de l'horodatage de l'indicateur tactile.

Dans un mode d'exécution, l'instruction d'interaction comprend un identifiant cible et la donnée respiratoire est générée en fonction de l'identifiant cible et de l'identifiant de l'indicateur tactile. Un avantage est de pouvoir intégrer dans la donnée respiratoire le temps de réaction et/ou les capacités de synchronisation des muscles respiratoires de l'utilisateur. L'avantage est de donner des consignes d'interaction avec une touche particulière et détecter si la bonne touche a bien été appuyée.

Dans un mode d'exécution, la méthode comprend en outre une mesure préalable physiologique de l'utilisateur et comprend l'émission d'une alerte si ladite mesure physiologique est dehors d'une plage de valeurs prédéfinie. Un avantage de ce mode est d'empêcher l'utilisateur de faire les exercices respiratoires si son état ne le permet pas ou présente un risque.

Dans un mode d'exécution, la mesure physiologique comprend une fréquence cardiaque et/ou un taux de saturation en oxygène dans le sang mesurés par un oxymètre à réflexion arrangé sur la surface de l'unité de respiration. Un avantage est d'empêcher l'utilisateur d'utiliser le dispositif si son taux d'oxygène dans le sang est trop faible. En effet, les exercices respiratoires auraient tendance à faire baisser cette valeur, présentant alors un risque si le taux de l'utilisateur était déjà trop bas avant le début de l'exercice. Un autre avantage est de surveiller un utilisateur pendant un exercice. L'utilisateur peut alors continuer à s'entrainer en toute confiance, car il sait qu'une alerte interviendra dans le cas où ses mesures physiologiques seraient trop basses.

Dans un mode d'exécution, la méthode comprend en outre la détermination d'un score en fonction de l'indicateur respiratoire et de l'indicateur tactile ou en fonction de la donnée respiratoire. Un avantage est d'obtenir une donnée reproductible permettant de suivre sur le long terme le score de l'utilisateur pour voir sa progression. Un autre avantage est de pouvoir se comparer à d'autres utilisateurs.

Dans un mode d'exécution, la méthode comprend, en outre, l'affichage d'une image d'un jeu vidéo interactif sur un afficheur, le jeu vidéo interactif incluant un élément contrôlable en fonction de l'indicateur respiratoire et de l'indicateur tactile ou en fonction de la donnée respiratoire. Un avantage est de pouvoir transposer les consignes dans un jeu vidéo permettant à l'utilisateur de moins s'ennuyer en réalisant les exercices. Un avantage est donc d'améliorer l'observance de l'utilisateur dans le traitement de sa maladie chronique respiratoire.

Selon un autre aspect, l'invention concerne un dispositif, de génération d'une donnée respiratoire d'un utilisateur comprenant une unité de respiration. L'unité de respiration comprend un capteur de pression d'air pour mesurer la pression d'air expiré et/ou inspiré par l'utilisateur et au moins deux interfaces tactiles solidaires de l'unité de respiration.

Selon un aspect alternatif, l'invention concerne un dispositif de génération d'une donnée respiratoire d'un utilisateur comprenant une unité de respiration et au moins deux interfaces tactiles. L'unité de respiration comprend un capteur de pression d'air pour mesurer la pression d'air expiré et/ou inspiré par l'utilisateur. Les interfaces tactiles peuvent être agencées sur une manette déportée.

Le dispositif selon l'un ou l'autre aspect comprend également un terminal électronique comprenant un calculateur. Dans un mode de réalisation, le calculateur adapté pour exécuter les étapes de la méthode selon l'invention. Le dispositif comprend alors des moyens de connexions pour connecter l'unité de respiration et/ou les interfaces tactiles au terminal électronique. Un avantage est de permettre à l'utilisateur d'interagir sur des interfaces tactiles agencées sur le même objet dans lequel il respire. L'utilisateur n'a ainsi qu'un seul objet à tenir. Un avantage est de faciliter des exercices comprenant des instructions de respiration et d'interactions tactiles simultanées. Un avantage des interfaces tactiles déportées de l'unité de respiration est de pouvoir reproduire la sensation d'une manette de jeu pour l'utilisateur. Un autre avantage est de pouvoir ainsi interagir plus facilement sans avoir à monter les bras pour tenir l'unité de respiration.

Dans un mode de réalisation, le dispositif comprend deux manettes non solidaires l'une de l'autre et comprenant chacune au moins une interface tactile telle que décrite ci-dessus. Un avantage est de pouvoir prendre une manette dans chaque main et de pouvoir ainsi interagir plus facilement en gardant une pleine mobilité des bras.

Dans un mode de réalisation, le dispositif comprend un moyen de communication connecté au terminal électronique pour transmettre des données à l'utilisateur. Un avantage est d'embarquer le terminal électronique dans un appareil distant connecté à l'unité de respiration et/ou aux interfaces tactiles.

Dans un mode de réalisation, le dispositif comprend des moyens lumineux conçus pour être allumés en fonction de l'indicateur respiratoire et/ou de l'indicateur tactile.

Dans un mode de réalisation, le dispositif comprend un oxymètre à réflexion sur la surface de l'unité de respiration. Un avantage est de permettre la mesure du taux d'oxygène dans le sang de l'utilisateur avant ou pendant les exercices par simple pression du doigt sur une portion de la surface de l'unité de respiration. L'utilisateur n'a pas besoin de réaliser une action particulière pour prendre ce genre de mesure et n'a pas à s'équiper d'un dispositif additionnel encombrant.

Dans un mode de réalisation, le dispositif comprend un moyen d'affichage. Le moyen d'affichage est connecté au terminal électronique et/ou au calculateur. Un avantage est de permettre l'affichage des consignes multimédia à l'utilisateur pendant un exercice. Un autre avantage est de permettre l'affichage d'un score ou d'un jeu vidéo interactif à l'utilisateur.

Dans un mode de réalisation, l'unité de respiration comprend en outre au moins un capteur de mouvement pour mesurer un angle d'inclinaison de ladite unité de respiration. Le capteur de mouvement est préférentiellement connecté au terminal électronique. Le capteur de mouvement permet de fournir des informations de mouvement angulaire de l'unité de respiration. Un premier avantage est d'intégrer dans la consigne multimédia une consigne de déplacement angulaire pour détourner l'attention de l'utilisateur afin d'entrainer son souffle sans avoir la pleine attention de l'utilisateur sur son souffle.

Dans un mode de réalisation, le terminal électronique est configuré pour déterminer l'orientation de l'unité de respiration à partir des données fournies par le capteur de mouvement. Le terminal électronique peut comprendre l'affichage d'une orientation cible prédéterminée de l'unité de respiration et la génération et l'affichage d'un indicateur en fonction de l'orientation de l'unité de respiration par rapport à l'orientation cible prédéterminée. Un avantage est de guider un utilisateur sur l'orientation de l'unité de respiration. Par exemple, le dispositif peut être utilisé pour l'administration d'un agent thérapeutique nécessitant une orientation de l'unité de respiration particulière. Dans un autre exemple, le dispositif peut être avantageusement utilisé pour simuler l'administration d'un agent thérapeutique.

Selon un autre aspect, l'invention concerne un produit programme d'ordinateur comprenant des instructions qui conduisent le dispositif selon l'invention à exécuter les étapes de la méthode selon l'invention.

Selon un aspect, l'invention concerne un produit programme d'ordinateur comprenant des instructions qui, lorsque le programme est exécuté par un ordinateur, conduisent l'ordinateur à mettre en oeuvre la méthode selon l'invention. Le produit programme d'ordinateur peut comprendre des instructions qui, lorsque le programme est exécuté par le calculateur du terminal électronique du dispositif selon l'invention, conduisent ledit calculateur à mettre en oeuvre la méthode selon l'invention.

Selon un dernier aspect, l'invention concerne un support lisible par ordinateur, sur lequel est enregistré le programme d'ordinateur selon l'invention. Préférentiellement le dispositif selon l'invention comprend une telle mémoire ou comprend des moyens de connexion à une telle mémoire.

### Brève description des figures

D'autres caractéristiques et avantages de l'invention ressortiront à la lecture de la description détaillée qui suit, en référence aux figures annexées, qui illustrent :
La figure 1 est une vue schématique d'un dispositif selon un mode de réalisation de l'invention dans lequel le dispositif comprend un embout buccal monté sur une unité de respiration comprenant des boutons d'interactions et une sonde ; l'unité de respiration étant connectée à un appareil distant pour le traitement des données et comprenant un afficheur pour interagir avec l'utilisateur.
La figure 2A est une vue en coupe schématique d'une unité de respiration et d'un embout buccal amovible selon un mode de réalisation de l'invention comprenant une chambre atmosphérique et une chambre fluidique d'expiration connectée à une entrée sur laquelle peut être monté un embout buccal. La chambre atmosphérique et la chambre fluidique d'expiration comprennent chacune au moins un capteur de pression d'air relié à un circuit imprimé. L'unité de contrôle comprend des boutons d'interactions connectés au circuit imprimé.
La figure 2B est une vue en coupe schématique d'une unité de respiration et d'un embout buccal amovible selon un mode de réalisation de l'invention dans lequel la chambre fluidique d'expiration est agencée dans l'embout buccal amovible.
La figure 3 est une vue de coupe schématique d'un bouton d'interaction de l'unité de respiration.
La figure 4 est une vue de l'affichage d'un jeu vidéo selon un mode d'exécution de l'invention.
La figure 5 est un logigramme schématique de la méthode selon un mode d'exécution de l'invention.
La figure 6 est une vue en perspective de trois embouts buccaux amovibles différents.
La figure 7 est une vue en perspective d'un mode de réalisation d'une unité de respiration sur laquelle est monté un embout.
La figure 8 est une vue en perspective d'une unité de respiration selon la figure 7 sans embout. L'unité de respiration comprend deux sorties pour coopérer avec des moyens de connexions de l'embout. Les sorties sont connectées fluidiquement à des capteurs de pression de l'unité de respiration. Une sortie est destinée à être connectée à une sortie fluidique de l'embout pour recevoir la bouche du sujet et une autre sortie est destinée à être connectée à une sortie fluidique de l'embout pour mesurer la pression atmosphérique lorsque l'utilisateur insère l'embout dans la bouche.

### Description de l'invention

Dans la présente description, on appelle « inspiration » la phase de respiration pendant laquelle l'air atmosphérique pénètre dans les poumons. On appelle « expiration » la phase de respiration pendant laquelle l'air est expulsé hors des poumons.

Selon un premier aspect, l'invention concerne un dispositif 100 de génération d'une donnée respiratoire. Le dispositif 100 de génération d'une donnée respiratoire comprend une unité de respiration 1. Le dispositif 100 peut comprendre également un terminal électronique 10.

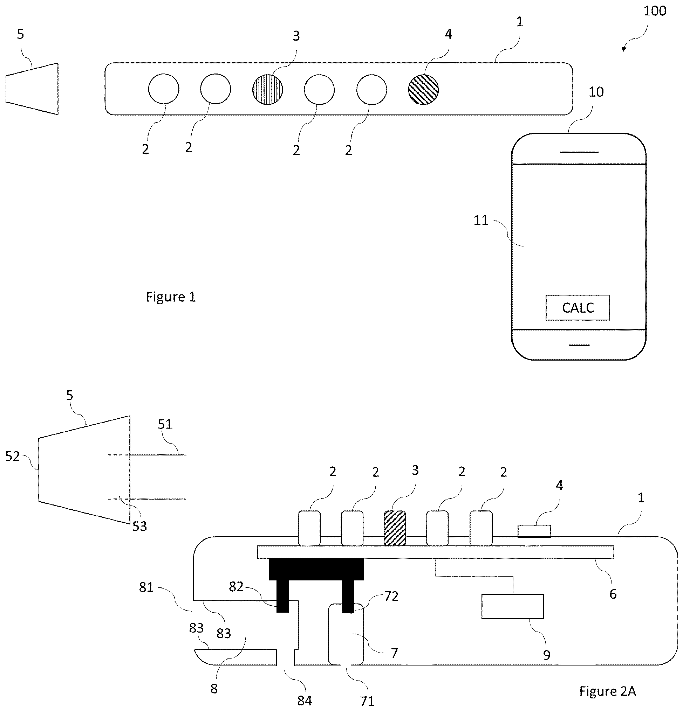

Un premier exemple d'unité de respiration 1 est illustré sur la figure 2A. L'unité de respiration 1 comprend une chambre fluidique d'expiration 8 pour recevoir l'air expiré par un utilisateur.

L'unité de respiration 1 peut comprendre un embout buccal 5 dans lequel l'utilisateur peut placer les lèvres pour expirer et inspirer.

L'embout buccal 5 comprend une ouverture 52. L'embout buccal 5 comprend une cavité 53 permettant de relier fluidiquement l'ouverture 52 au corps principal de l'unité de respiration, notamment à la chambre fluidique d'expiration 8. À ce titre, l'embout comprend un moyen de connexion 51 à un moyen de connexion 83 complémentaire de l'unité de respiration.

Par « chambre fluidique », on désigne toute structure capable d'accueillir un volume de fluide pour la mesure de la pression dans une telle structure. Une chambre fluidique peut à ce titre comprendre une portion d'un canal fluidique.

Préférentiellement, l'embout buccal 5 est fixé à l'unité de respiration de manière amovible.

La chambre fluidique d'expiration 8 comprend une ouverture fluidique 81. L'ouverture fluidique peut être en communication fluidique avec la cavité 53 de l'embout buccal 5. L'air des inspirations et des expirations de l'utilisateur passe alors par ladite chambre fluidique, créant respectivement une dépression et une surpression mesurables par un capteur de pression d'air.

Un avantage est de pouvoir retirer l'embout buccal 5 pour pouvoir le nettoyer sans nettoyer toute l'unité de respiration. Un autre avantage est de permettre le remplacement de l'embout buccal 5 pour adapter l'embout buccal à l'utilisateur. L'unité de respiration est alors adaptée pour être utilisée par différentes personnes ayant une anatomie buccale différente, notamment en changeant la taille de l'embout buccal amovible 5. Un autre avantage est de pouvoir adapter l'embout buccal à un exercice particulier, par exemple, un exemple où l'utilisateur doit expirer avec les lèvres pincées.

Dans un mode de réalisation non représenté, la chambre fluidique d'expiration 8 comprend une membrane. La membrane est préférentiellement imperméable à l'air et permet de créer une séparation étanche entre le capteur de pression 82 et l'environnement extérieure. La membrane est déformable de manière à propager la pression d'air subie par la membrane à l'air présent dans le canal fluidique 87 entre la membrane et le capteur de pression d'air 82. Un avantage est de protéger le canal fluidique et le capteur de pression d'air des contaminations par des poussières, des impuretés, des bactéries ou des virus.

La membrane peut être agencé au niveau de l'ouverture fluidique 81 ou au niveau du canal fluidique 87 ou de manière générale entre l'ouverture fluidique 81 et le capteur de pression d'air 82. La membrane peut être réalisée en matériau plastique tel qu'un matériau élastomère.

Lorsque la chambre fluidique d'expiration 8 comprend une membrane, le capteur de pression d'air permet alors de mesurer la pression d'air expiré et/ou inspiré par l'utilisateur dans la chambre fluidique d'expiration. En effet, la portion de la chambre fluidique entre le capteur de pression d'air et la membrane comprend une pression variable en fonction de la pression d'air expiré et/ou inspiré par l'utilisateur grâce à la déformation de la membrane.

La chambre fluidique d'expiration 8 est formée par des parois 83. Au moins une des parois comprend un trou de sortie d'air 84. Le trou de sortie d'air 84 est en communication fluidique avec l'extérieur de l'unité de respiration 1 et permet l'échappement des gaz de la chambre fluidique 8. Préférentiellement, le trou de sortie d'air 84 est agencé transversalement ou sensiblement perpendiculairement à la direction des gaz dans la chambre fluidique par l'ouverture 81. Préférentiellement, la section du trou de sortie 84 est inférieure à la section de l'ouverture 81 de la chambre fluidique d'expiration 8. Cet arrangement permet de créer une résistance à la sortie de l'air et créer une pression dans la chambre fluidique d'expiration 8 qui pourra être mesurée, préférentiellement lors de l'inspiration et/ou lors de l'expiration de l'utilisateur. Ce trou de sortie d'air 84 peut également être conçu pour permettre à l'air dans le canal fluidique entre l'ouverture de l'embout 52 et le capteur 82 de s'échapper. Il permet alors avantageusement à l'utilisateur des temps de souffle plus long sans créer de surpression expiratoire dans le canal fluidique.

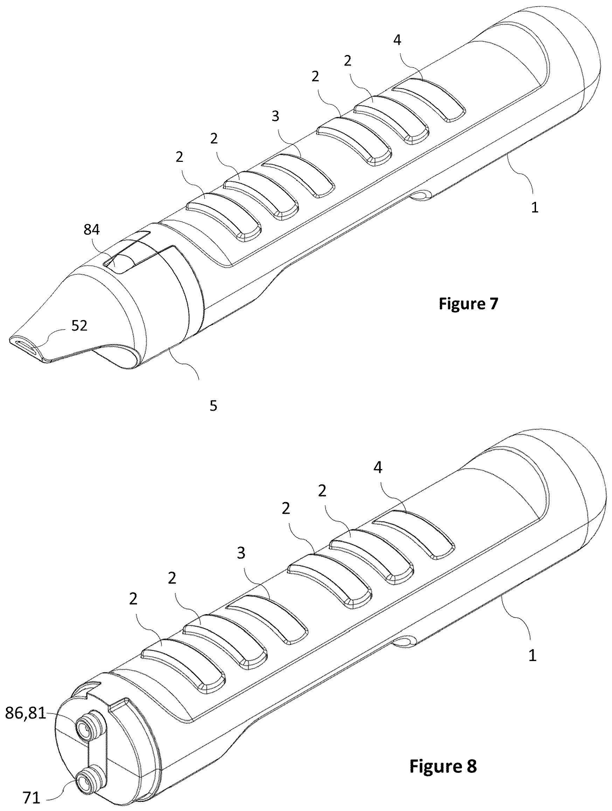

Dans un mode de réalisation, le trou de sortie d'air 84 est agencé sur l'embout 5 comme illustré sur la figure 7.

L'unité de respiration 1 permet la mesure de la pression d'air dans la chambre fluidique d'expiration 8. L'unité de respiration 1 peut, à ce titre, comprendre un capteur de pression d'air 82.

Le capteur de pression d'air est préférentiellement constitué ou comprend un élément sensible à la pression pour déterminer une pression réelle appliquée au capteur pour convertir cette information en signal de sortie. Le capteur de pression est connecté au terminal électronique de manière à transmettre au terminal électronique le signal de sortie.

Le capteur de pression peut comprendre un élément sensible à la pression sur lequel une jauge de pression est collée ou appliquée par pulvérisation. Cet élément sensible à la pression peut comprendre un diaphragme.

Le capteur de pression d'air peut comprendre également un capteur de pression capacitif ou un capteur de pression piézorésistif bien connu de l'homme du métier.

Le capteur de pression d'air 82 peut être agencé contre une paroi 83 de la chambre fluidique d'expiration 8. Le capteur de pression 82 peut être agencé dans un canal 87 aveugle en communication fluidique avec la chambre fluidique d'expiration 8.

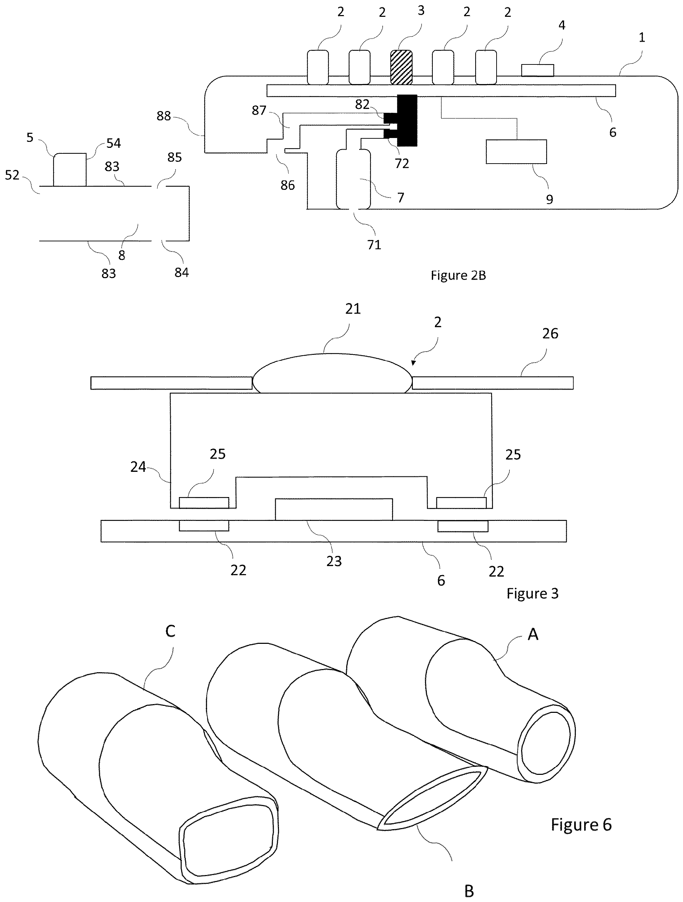

Dans un second exemple illustré sur la figure 2B, l'embout buccal 5 comprend la chambre fluidique d'expiration 8. L'embout buccal 5 comprend une ouverture d'entrée 52 par laquelle l'utilisateur peut inspirer et expirer. L'embout buccal est amovible. La chambre fluidique d'expiration 8 comprend une deuxième ouverture de sortie fluidique 84 et une troisième ouverture 85. Préférentiellement, la deuxième ouverture 84 et la troisième ouverture 85 sont agencées sur deux parois 83 opposées de la chambre fluidique d'expiration 8.

Un avantage de placer la chambre fluidique d'expiration 8 dans l'embout buccal amovible 5 est de faciliter le nettoyage de ladite chambre et d'éviter l'accumulation d'humidité.

Dans ce mode réalisation, l'unité de respiration 1 comprend un évidement conçu pour recevoir l'embout buccal amovible 5. Dans ce cas, l'unité de respiration 1 comprend des moyens de coopération 88 avec l'embout buccal amovible 5. Préférentiellement, l'embout buccal amovible 5 comprend des moyens de coopération 54 complémentaires pour être fixé de manière amovible à l'unité de respiration 1.

L'unité de respiration 1 peut comprendre un canal fluidique 87 aveugle (c'est-à-dire non-débouchant) s'étendant depuis une entrée 86. Le canal fluidique est dans ce cas une continuité fluidique de la chambre fluidique d'expiration 8. Le canal fluidique comprend un capteur de pression d'air 82. Le capteur de pression d'air 82 permet de mesurer la pression d'air expiré par l'utilisateur. Le capteur de pression d'air 82 permet de mesurer la pression d'air dans la chambre fluidique d'expiration 8. L'entrée 86 du canal fluidique 87 est arrangée pour coopérer avec la troisième ouverture de sortie fluidique 85 de la chambre fluidique d'expiration de l'embout buccal lorsque ledit embout buccal est monté sur l'unité de respiration 1.

Un avantage du canal fluidique 87 est de protéger le capteur de pression d'air 82, notamment lors de la manipulation de l'unité de respiration quand l'embout buccal est retiré.

L'ouverture de sortie 84 permet à l'air expiré de s'échapper de la chambre fluidique d'expiration 8 vers l'extérieur de l'unité 1. L'utilisateur peut alors expirer ou inspirer en continu dans la chambre fluidique d'expiration 8. L'intérêt de cette ouverture de sortie 84 est également de créer une résistance à l'entrée et à la sortie de l'air dans la chambre fluidique respectivement lors de l'inspiration et lors de l'expiration. Cette résistance permet avantageusement d'augmenter la pression causée par l'inspiration et/ou l'expiration dans la chambre fluidique d'expiration.

Dans un mode de réalisation, l'unité de respiration 1 comprend une chambre fluidique atmosphérique 7. Cette chambre fluidique atmosphérique permet de mesurer la pression atmosphérique. Un avantage est de pouvoir fournir une mesure de la pression atmosphérique pour servir de référentiel à la mesure de pression de l'air dans la chambre fluidique d'expiration 8. La chambre fluidique atmosphérique 7 comprend un second capteur de pression de l'air 72 et un trou de sortie 71 en communication fluidique avec l'environnement extérieur, optionnellement à travers un canal de l'embout 5. Le second capteur de pression de l'air 72 peut comprendre un capteur différentiel de pression. Le second capteur de pression d'air 72 est ainsi avantageusement protégé. Dans un mode représenté sur la figure 8, le trou de sortie 71 est agencé de manière à être connecté à un canal fluidique ouvert de l'embout. Un avantage est d'agrandir la chambre fluidique atmosphérique 7 afin d'améliorer la précision de la pression atmosphérique mesurée.

L'embout 5 peut comprendre une seconde ouverture, une seconde cavité, un second moyen de connexion, pour opérer une connexion avec le trou 71 de la chambre fluidique atmosphérique 7.

Dans un mode de réalisation, la chambre fluidique atmosphérique 7 comprend également une membrane telle que celle décrite pour la chambre fluidique d'expiration 8.

Dans un mode de réalisation, l'embout 5 comprend un spiromètre. Le trou de sortie 71 peut être connecté à la pression expiratoire de l'utilisateur dans le spiromètre. Le spiromètre peut comprendre un cylindre creux dans lequel s'écoule le flux d'air expiré ou inspiré par l'utilisateur. Dans ce mode, le trou de sortie 71 est connecté à une première section dudit cylindre et l'ouverture 81 de la chambre fluidique d'expiration est connectée à une seconde section du cylindre différente de la première section. De cette manière, chaque capteur de pression d'air mesure une pression relative à la vitesse d'écoulement de l'air expiré et/ou inspiré au niveau de différentes sections du cylindre.

Le terminal électronique peut être configuré pour générer un indicateur illustrant la capacité pulmonaire de l'individu en fonction de la différence des pressions mesurées par les deux capteurs de pression d'air 82, 72.

Dans un mode de réalisation, le dispositif 100 comprend une pluralité d'interfaces tactiles 2. Préférentiellement, les interfaces tactiles 2 permettent de détecter une interaction d'un utilisateur. Dans un premier mode de réalisation, les interfaces tactiles 2 comprennent des boutons. Un bouton est illustré en vue de coupe sur la figure 3. Le bouton 2 comprend un capuchon mobile 21 sur lequel l'utilisateur peut appuyer avec son doigt. Lors de l'appui, le capuchon 21 est déplacé le long d'une course prédéfinie, préférentiellement dans une direction sensiblement perpendiculaire à la surface 26 de l'unité de respiration 1. Le déplacement du capuchon 21 peut provoquer le déplacement d'une pièce de connexion 24.

Le bouton 2 peut comprendre un interrupteur dont l'état est modifié en fonction de la course de la pièce de connexion 24. Préférentiellement, l'interrupteur est connecté à un circuit imprimé 6.

Comme illustré sur la figure 3, la pièce de connexion 26 peut comprendre au moins une piste de connexion 25. La piste de connexion 25 est arrangée pour entrer en contact avec une piste de connexion 22 du circuit imprimé 6 lorsque la pièce de connexion 26 est en bout de course. Le contact entre les deux pistes de connexion 25, 22 est détecté et permet la détection d'une interaction sur l'interface utilisateur.

Dans d'autres modes de réalisation, les interfaces peuvent comprendre des surfaces tactiles, des trous comprenant des moyens pour la détection d'une interaction de l'utilisateur sur lesdites interfaces. Les interfaces 2 peuvent également comprendre des capteurs de pression pour mesurer la pression exercée par l'utilisateur lors d'une interaction.

Dans un mode de réalisation, les interfaces tactiles 2 sont solidaires de l'unité de respiration 1. Dans ce cas, les interfaces tactiles 2 sont sur le même objet que celui dans lequel l'utilisateur expire et dans lequel la pression d'air est mesurée. Un avantage est de permettre à l'utilisateur d'expirer et d'interagir avec les interfaces tactiles simultanément.

Dans les exemples illustrés sur les figures 1 et 7, l'unité de respiration 1 a une forme de flute et les interfaces tactiles 2 sont disposées de la même manière que sont agencées les touches d'une flute. L'unité de respiration 1 peut comprendre quatre interfaces tactiles 2. Les quatre interfaces tactiles peuvent être alignées les unes avec les autres et avec l'ouverture 52, 81 de la chambre fluidique d'expiration. Dans d'autres modes de réalisation non représentés, l'unité de respiration 1 peut présenter une forme de saxophone, ou de tout autre type d'instruments ou d'instruments à vent. Dans tous les cas, les interfaces tactiles 2 sont agencées pour être accessibles aux doigts de l'utilisateur de la même manière qu'un instrument à vent.

Préférentiellement, les interfaces tactiles 2 sont positionnées sur la surface 26 de l'unité de respiration 1 pour être chacune accessibles par un doigt différent de l'utilisateur lorsque celui-ci respire dans l'embout buccal 5.

Dans un mode de réalisation alternatif non représenté, les interfaces tactiles sont agencées à la surface d'un autre objet différent que l'unité de respiration. Par exemple, les interfaces tactiles peuvent être agencées à la surface d'une manette conçue pour être tenue par la main de l'utilisateur. Le dispositif peut comprendre deux manettes, chacune comprenant au moins une interface tactile. L'interface tactile peut être connecté au terminal électronique par une connexion filaire ou une connexion sans-fil telle qu'une connexion Bluetooth, Wi-Fi, ou tout autre moyen de connexion sans-fil connu de l'homme du métier.

Chaque interface tactile 2 peut être connectée à un circuit imprimé 6. Le dispositif 100 est alors configuré pour détecter une interaction sur une interface tactile.

Préférentiellement, l'unité de respiration 1 peut comprendre des moyens d'émission de lumière 23. Les moyens d'émission de lumière 23 peuvent comprendre une ampoule ou une diode électroluminescente. L'unité de respiration peut être conçue pour que les moyens d'émission de lumière 23 s'allument lorsqu'une interaction est détectée sur une interface. Préférentiellement, chaque moyen d'émission de lumière 23 est associé à une interface et l'unité est configurée pour que chaque moyen d'émission de lumière 23 s'allume lorsqu'une interaction est détectée sur l'interface tactile 2 associée. Dans le mode de réalisation illustré sur la figure 3, la pièce de connexion 24 est transparente à la lumière. La pièce de connexion 24 permet avantageusement de laisser passer la lumière émise par le moyen d'émission de lumière 23. Dans un mode de réalisation, le dispositif est configuré pour allumer les moyens d'émission de lumière pour transmettre une information à l'utilisateur tels qu'un niveau faible de batterie, l'état d'une connexion entre l'unité de respiration 1 et le terminal électronique 10.

Dans un mode de réalisation, l'unité de respiration 1 comprend un moyen de mesure de données physiologiques 3 de l'utilisateur. Préférentiellement, le moyen de mesure de données physiologiques 3 est un moyen tactile. Le moyen de mesure 3 peut être agencé à la surface 26 de l'unité de respiration. Le moyen de mesure 3 est préférentiellement solidaire de la surface 26 de l'unité de respiration.

Le moyen de mesure comprend préférentiellement un photopléthysmographe à réflexion ou un oxymètre colorimétrique à réflexion. Un photopléthysmographe ou un oxymètre colorimétrique permet de quantifier la saturation en oxygène de l'hémoglobine au niveau des capillaires sanguins ainsi que de mesurer la fréquence cardiaque de l'utilisateur.

Préférentiellement, le moyen de mesure 3 comprend un émetteur de lumière et un capteur de la lumière réfléchie par l'utilisateur, préférentiellement par le doigt de l'utilisateur. L'émetteur et le capteur sont donc placés sur la même surface. Un avantage de capter la lumière réfléchie plutôt que la lumière transmise est que l'utilisateur n'a pas à réaliser d'action particulière propre à la mesure de la saturation pulsée en oxygène. L'utilisateur a juste à poser son doigt sur le moyen de mesure 3 pour obtenir une mesure physiologique. L'utilisateur peut avantageusement utiliser le dispositif 100 et prendre des mesures pendant un exercice sans avoir à réaliser des manipulations pouvant freiner l'exercice et sans gêner la liberté de ses doigts par un dispositif de pincement.

Dans un exemple illustré sur les figures 1, 2A et 2B, l'unité de respiration 1 comprend 4 interfaces tactiles et comprend un moyen de mesure 3 agencé entre 2 interfaces tactiles.

Dans un autre exemple non représenté, le moyen de mesure 3 peut être agencé en tout endroit de l'unité de respiration 1 susceptible d'être accessible par un doigt de l'utilisateur lorsqu'il tient l'unité de respiration. Par exemple, le moyen de mesure peut être agencé sur la face opposée de la face comprenant les interfaces tactiles 2. Un avantage est de permettre à l'utilisateur de poser son pouce sur l'interface tactile. Par sa plus grande surface de contact et son volume plus important, la mesure de données physiologiques par le pouce de l'utilisateur est avantageusement plus fiable et/ou précise.

Dans un mode de réalisation, l'unité de respiration comprend un pad 4. Le pad est agencé à proximité des interfaces tactiles 2. Le pad 4 est préférentiellement agencé pour reposer un doigt de l'utilisateur.

Un avantage du pad 4 est de tenir l'unité de respiration de manière équilibrée avec au moins un doigt de l'utilisateur. En effet, dans l'exemple d'une unité de respiration en forme de flute, les deux pouces de l'utilisateur sont agencés en dessous de l'unité. Le majeur, l'index et l'annulaire sont posés sur le dessus de l'unité de respiration. Le pad 4 permet avantageusement à l'utilisateur de maintenir l'équilibre de l'unité de respiration 1, notamment sans risque d'interagir par erreur avec une interface tactile 2.

Le pad 4 peut comprendre une surface rugueuse ou adhérente telle qu'une surface comprenant un silicone ou des picots. L'unité de respiration 1 peut comprendre deux ou plus pad 4, Ceci permet avantageusement de reposer les doigts qui ne sont pas utilisés pour faire interface avec les interfaces tactiles 2 et/ou le moyen de mesure 3.

Le dispositif 100 selon l'invention comprend un terminal électronique 10. Le terminal électronique 10 permet de recevoir et de traiter les données des différents capteurs de l'unité de mesure tels que le ou les capteurs de pression d'air 82, 72, les interfaces tactiles 2 et/ou le moyen de mesure 3.

Le terminal électronique 10 peut être intégré dans l'unité de mesure. Dans un autre exemple illustré sur la figure 1, le terminal électronique 10 est embarqué dans un appareil distant. Dans cet exemple, l'unité de respiration 1 comprend un transmetteur 9. Le transmetteur 9 est connecté aux différents capteurs 82, 72, 3, 2 de l'unité de respiration 1 pour recevoir et transmettre les données mesurées ou détecter au terminal électronique 10 de l'appareil distant. Dans un exemple illustré sur les figures 2A et 2B, l'unité de respiration 1 comprend un circuit imprimé 6 connecté aux différents capteurs 82, 72, 2, 3 et le circuit imprimé 6 est connecté au transmetteur 9. Le circuit imprimé 6 permet avantageusement la récupération et/ou le traitement des différentes mesures et détection pour les transmettre au transmetteur 9 et/ou au terminal électronique 10.

Le terminal électronique peut également être configuré pour recevoir des informations propres à l'unité de respiration 1. Le dispositif peut être configuré pour que le terminal électronique puisse recevoir des informations relatives à l'état de la batterie de l'unité de respiration ou relatives à un taux d'humidité dans la chambre fluidique d'expiration 8.

Le dispositif 100 peut également comprendre un moyen de communication 11. Le moyen de communication 11 permet de transmettre des informations à l'utilisateur. Le moyen de communication 11 peut comprendre un transmetteur sonore ou un afficheur. Le terminal électronique 10 est connecté audit moyen de communication 11. Le moyen de communication 11 permet de transmettre à l'utilisateur des variables mesurées par le dispositif 100 à partir des mesures du capteur de pression d'air 82 et/ou des interactions tactiles détectées par les interfaces tactiles 2. Dans un mode de réalisation, le moyen de communication comprend les moyens d'émission de lumière 23 de l'unité de respiration 1.

Le terminal électronique 10 comprend un calculateur CALC.

Le terminal électronique 10 peut comprendre une mémoire, préférentiellement une mémoire non transitoire.

Le terminal électronique 10 permet avantageusement de traiter les signaux réceptionnés.

Selon un mode de réalisation, le dispositif 100 comprend au moins deux embouts buccaux amovibles 5 différents. Les différents embouts buccaux différents notamment par la surface et la forme de la section de leur ouverture 52. Trois types d'embouts sont décrits ci-dessous en référence à la figure 6.

Un premier embout buccal amovible A comprend une ouverture 52 de section cylindrique ou sensiblement cylindrique. Le premier embout permet avantageusement de maximiser le débit d'air. En effet, une telle section permet d'augmenter le débit d'air expiré par l'utilisateur. Un avantage est de permettre la mesure de constantes par le dispositif telle que la capacité pulmonaire, le volume expiratoire maximal par seconde, le débit expiratoire de pointe. Préférentiellement, la section de l'ouverture du premier embout buccal amovible A est comprise entre 900 mm² et 600 mm² lorsqu'il est destiné à un adulte ou entre 300 mm² et 420 mm² lorsqu'il est destiné à un enfant. La section de l'ouverture du premier embout buccal amovible A peut comprendre une ouverture dont le diamètre de la section est compris entre 35 et 25 mm ou entre 25 et 20 mm.

Dans un mode de réalisation, le premier embout buccal amovible A comprend une périphérie conçue pour recevoir par enfoncement une tête d'embout à usage unique (non représentée). La tête d'embout peut comprendre une portion cylindrique conçue pour coopérer avec le premier embout buccal amovible A. La tête d'embout peut comprendre n'importe quelle forme lui permettant de coopérer avec l'embout amovible 5.

Un deuxième embout buccal amovible B comprend une ouverture de section de préférence ovoïdale ou sensiblement ovoïdale. Préférentiellement, la section de l'ouverture du deuxième embout 8 est comprise entre 150 mm² et 550 mm² ou entre 300 mm² et 400 mm². Le deuxième embout permet avantageusement à l'utilisateur d'effectuer les exercices en expiration prolongée et profonde.

Un troisième embout buccal amovible C permet à l'utilisateur de réaliser des exercices pour favoriser l'expectoration de mucus des poumons de l'utilisateur. Le troisième embout comprend un moyen de pression expiratoire positive oscillant. Le moyen de pression expiratoire positive oscillant provoque des pulsions de résistance lors de l'expiration de l'utilisateur. La résistance créer une accumulation de pression positive dans les poumons de l'utilisateur ce qui aide à garder ouvertes les voies respiratoires. De plus, les pulsions créent des vibrations à l'intérieur des voies respiratoires, pour aider à amincir et déloger le mucus qui serait trop épais ou collant pour être délogé par la seule force de la pression. L'action combinée de la pression et des oscillations permet de faire avancer le mucus vers les voies respiratoires centrales, d'où il peut être expulsé par la toux.

Ainsi, le dispositif 100 selon un mode de réalisation de l'invention comprend au moins un embout ou au moins deux embouts parmi le premier, le deuxième et le troisième embout buccal amovible.

Préférentiellement, le dispositif comprend une unité de respiration et les trois embouts buccaux amovibles : le premier, le deuxième et le troisième. Naturellement, d'autres types d'embouts buccaux amovibles peuvent être imaginés et intégrés au dispositif 100, notamment pour la réalisation d'exercices spécifiques.

Un avantage de ces trois embouts buccaux amovibles 5 est de pouvoir, avec une unique unité de respiration 1, obtenir trois fonctions d'utilisation en remplaçant uniquement l'embout buccal amovible 5. L'unité de respiration 1 peut ainsi être utilisée pour une fonction de mesure de constantes avec le premier embout buccal amovible A, pour une fonction d'entrainement à différents types d'expirations (expiration prolongée, expiration rapide) avec le deuxième embout buccal amovible B et pour une fonction d'assistance à l'expectoration (aussi appelé inhalothérapie) avec le troisième embout buccal amovible C.

Dans un mode de réalisation, le dispositif 100 comprend un identificateur de l'embout buccal amovible 5 qui est connecté à l'unité de respiration.

L'identificateur peut comprendre des moyens électroniques tels qu'un détecteur de pistes de connexion de l'embout buccal amovible.

L'identificateur peut comprendre des moyens de détection magnétiques. Ces moyens de détection magnétiques permettent de détecter l'embout buccal connecté à l'unité de respiration lorsque chacun des embouts buccaux amovibles comprend un moyen magnétique dont l'intensité magnétique et/ou la position sont différentes d'un embout à l'autre.

L'identificateur peut comprendre un moyen de détection lumineux d'un marqueur de l'embout buccal amovible. L'identificateur peut comprendre un moyen de détection mécanique. Par exemple, l'embout peut comprendre un insert mécanique spécifique conçu pour coopérer avec l'unité de respiration de manière à être reconnu ou identifié.

Un second aspect de l'invention concerne une méthode de génération d'une donnée respiratoire S d'un utilisateur.

Ladite méthode comprend préférentiellement l'utilisation d'un dispositif 100 selon le premier aspect de l'invention.

La méthode vise à générer une donnée respiratoire S quantifiant une performance respiratoire de l'utilisateur. La donnée respiratoire S est générée en fonction des mesures réalisées SI par le capteur de pression d'air 82 de la chambre fluidique d'expiration 8.

La méthode de génération d'une donnée respiratoire S vise à transmettre une consigne multimédia Cm à l'utilisateur et à mesurer la réponse de l'utilisateur suite à cette consigne. La donnée respiratoire S est ensuite générée à partir de cette réponse. Une réponse de l'utilisateur peut comprendre une expiration mesurable par le capteur de pression d'air 82 de la chambre fluidique d'expiration 8.

Un avantage est de permettre la transmission à l'utilisateur une consigne multimédia Cm et de générer la donnée respiratoire S en fonction de corrélation entre la consigne multimédia Cm et la réponse de l'utilisateur.

Dans un mode d'exécution de l'invention, la réponse peut comprendre une interaction SI avec une ou plusieurs des interfaces tactiles 2 de l'unité de respiration 1. L'utilisateur est alors invité à réaliser des exercices dans lesquels sont combinés des ordres d'expiration et/ou des ordres de mouvement des doigts. Un avantage est de permettre, par exemple, d'assister la régularité d'un rythme de respiration par le battement d'un rythme complémentaire ou analogue avec le doigt.

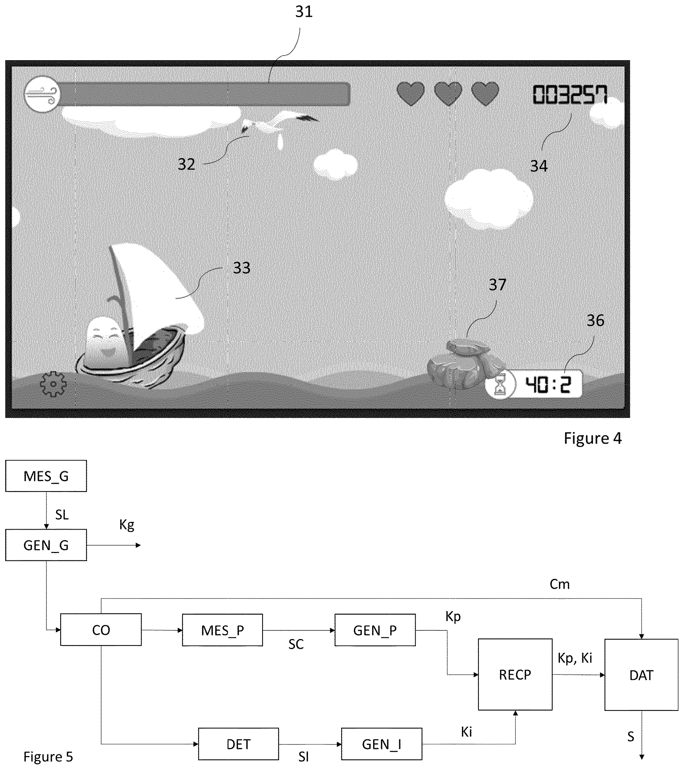

Un mode d'exécution de la méthode selon l'invention est décrit ci-dessous en référence à la figure 5.

Dans un mode de réalisation, la méthode comprend une mesure physiologique MES_G de l'utilisateur préalable à la génération de la donnée respiratoire et/ou préalable à la mesure SC de la pression d'air expirée par l'utilisateur.

La méthode peut comprendre la génération GEN_G et/ou l'émission d'une alerte Kg, lorsque la valeur physiologique mesurée est comprise dans une plage de valeurs prédéfinies ou en dehors d'une plage de valeurs prédéfinies.

La mesure physiologique SL comprend préférentiellement une mesure de la fréquence cardiaque de l'utilisateur. La mesure physiologique SL peut comprendre également une valeur de saturation en oxygène de l'hémoglobine de l'utilisateur.

Un avantage d'une telle mesure préalable est qu'elle permet à l'utilisateur d'utiliser le dispositif en toute sécurité. En effet, les utilisateurs souffrant d'insuffisance respiratoire peuvent voir une chute de l'oxygène dans le sang lors d'exercices de respiration. Cette mesure permet ainsi de détecter avant l'exercice si le taux de saturation en oxygène dans l'hémoglobine de l'utilisateur est suffisamment élevé pour permettre l'exercice en toute sécurité. Dans ce cas, un message d'alerte Kg peut être émis. Le message d'alerte Kg vise à déconseiller l'utilisateur de démarrer un entraînement avec le dispositif. Le message d'alerte Kg peut comprendre un message lumineux, par exemple par l'intermédiaire des moyens d'émission de lumière 23 de l'unité de respiration. Le message d'alerte Kg peut comprendre un message sonore. Le message d'alerte Kg peut comprendre un message affiché sur un afficheur. Le message d'alerte Kg peut comprendre un message sensoriel, par exemple, la vibration d'un vibreur agencé dans l'unité de respiration 1. Le message d'alerte Kg peut provoquer l'arrêt de l'unité de respiration ou bloquer son utilisation.

La mesure physiologique SL est préférentiellement mesurée avec le moyen de mesure 3 de l'unité de respiration 1 décrit ci-devant. Un avantage est de permettre à l'utilisateur d'effectuer une telle mesure par simple pose du doigt sur la surface 26 de l'unité de mesure. L'utilisateur n'a alors pas à déplacer ces mains entre la prise de la mesure physiologique et l'utilisation de l'unité de respiration.

Dans un mode d'exécution de l'invention, la méthode comprend l'arrêt de la méthode ou l'arrêt du dispositif, lorsque la valeur physiologique SL mesurée est comprise dans ou en dehors d'une première plage de valeur prédéfinie. Dans un mode d'exécution, la méthode comprend la génération d'une donnée respiratoire S lorsque la valeur physiologique mesurée est comprise dans ou en dehors d'une seconde plage de valeurs prédéfinie.

La méthode de génération d'une donnée respiratoire comprend la génération CO d'une consigne multimédia Cm. La consigne multimédia Cm générée est transmise à l'utilisateur. La consigne multimédia Cm peut être transmise à l'utilisateur par un afficheur 11 ou de manière sonore.

La consigne multimédia Cm peut comprendre des instructions d'expiration.

La génération CO de la consigne multimédia Cm est horodatée ou l'émission de la consigne multimédia est horodatée. L'horodatage de l'émission de la consigne multimédia permet avantageusement de comparer les dates d'émission de la consigne et de la réponse de l'utilisateur. Cette comparaison permet par exemple de calculer un écart entre une date cible et la date à laquelle l'utilisateur a effectué l'action (tactile et/ou respiratoire) demandée par la consigne multimédia.

La consigne multimédia Cm peut être optionnellement générée par un logiciel du terminal électronique 10.

La méthode de génération d'une donnée respiratoire comprend une mesure MEP_P d'une pression d'air SC expirée et/ou inspirée d'un utilisateur. La mesure MEP_P d'une pression d'air peut être mesurée par le capteur de pression d'air 82 de la chambre d'expiration 8 de l'unité de respiration 1 selon le premier aspect de l'invention.

Les mesures du capteur de pression d'air 82 de la chambre fluidique d'expiration 8 peuvent être enregistrées sur un support de stockage de données, notamment en temps réel.

Dans un mode d'exécution de l'invention, la mesure d'une pression d'air peut comprendre la mesure de l'air dans la chambre fluidique d'expiration pendant l'inspiration et/ou pendant l'expiration de l'utilisateur.

La méthode de génération d'une donnée respiratoire comprend la génération GEN_P d'un indicateur respiratoire Kp.

L'indicateur respiratoire Kp est généré en fonction de la pression d'air mesurée SC dans la chambre fluidique d'expiration 8.

L'indicateur respiratoire Kp est horodaté. La méthode peut comprendre une étape d'association de l'indicateur respiratoire à une date. Par « date », on entend une date et une heure. Cet horodatage a pour but d'enregistrer l'instant auquel la mesure et/ou la génération de l'indicateur respiratoire ont été réalisées.

L'indicateur respiratoire Kp peut être généré en fonction de la longueur d'une expiration, en fonction de la pression maximale sur une période de temps donnée.

Dans un mode d'exécution, la méthode comprend également une mesure de la pression d'air atmosphérique. Cette mesure est préférentiellement réalisée par le capteur de pression d'air 72 de la chambre fluidique atmosphérique 7 de l'unité de respiration 1 selon le premier aspect de l'invention. La mesure de pression d'air atmosphérique permet avantageusement de servir d'étalon à la pression d'air mesurée dans la chambre fluidique d'expiration. La mesure de la pression d'air SC dans la chambre fluidique d'expiration est alors indépendante de la pression d'air atmosphérique.

L'indicateur respiratoire Kp peut être généré en fonction de la pression d'air mesurée SC dans la chambre fluidique d'expiration et en fonction de la pression d'air atmosphérique mesurée.

La méthode de génération d'une donnée respiratoire peut comprendre la détection DET d'une interaction SI sur une interface tactile de l'unité de respiration 1. Une interaction est détectée lorsque l'utilisateur interagi avec au moins une interface tactile 2 de l'unité de respiration, par exemple par contact ou par pression avec le doigt sur au moins une interface tactile 2.

Un indicateur tactile Ki est généré GEN_P à partir ou en fonction de la détection d'une interaction. Par exemple, lorsque l'utilisateur appui sur un bouton de l'unité de respiration, un signal SI est généré. Le signal généré SI est transmis à un composant du circuit imprimé et/ou du terminal électronique. Le signal généré permet de détecter quelle interface a subi une interaction.

L'indicateur tactile Ki peut comprendre un identifiant de l'interface sur laquelle a été détectée une interaction, par exemple, un identifiant du bouton sur lequel une pression de l'utilisateur a été détectée. L'indicateur tactile Ki comprend un horodatage de la détection de l'interaction. L'indicateur peut comprendre une intensité de l'interaction, par exemple, le temps d'appui sur une interface tactile 2 ou la force avec laquelle l'utilisateur appuie sur l'interface tactile 2.

L'indicateur tactile Ki et/ou l'indicateur respiratoire Kp sont transmis à un calculateur CALC du terminal électronique 10. Comme décrit précédemment, le terminal électronique 10 peut être agencé dans l'unité de respiration 1 ou dans un appareil distant.

À partir de ces deux indicateurs horodatés et à partir de la consigne multimédia Cm horodatée, une donnée respiratoire S est générée. La donnée respiratoire S est préférentiellement générée par le calculateur CALC.

La donnée respiratoire S est générée en fonction de la corrélation des deux indicateurs Ki, Kp avec la consigne multimédia Cm. La donnée respiratoire S permet de quantifier une performance respiratoire de l'utilisateur. Par exemple, la donnée respiratoire S peut comprendre un score 34 dont la valeur augmente lorsque l'indicateur tactile Ki et l'indicateur respiratoire Kp sont conformes à la consigne multimédia Cm.

Dans un premier exemple, la consigne multimédia Cm peut comprendre des instructions de respiration et des instructions d'interaction tactile synchronisées. Les instructions de respiration comprennent des instructions d'expirations successives à au moins une date donnée, et préférentiellement de façon synchronisée, par exemple à un tempo donné. Les instructions d'interaction comprennent une consigne d'interaction avec les interfaces tactiles à au moins une date donnée, préférentiellement de façon synchronisée. Les instructions de respiration peuvent également comprendre des instructions d'inspiration datée. Les instructions peuvent être transmises à l'utilisateur et comprendre une ou plusieurs dates cibles d'expiration et une ou plusieurs dates cibles d'interaction tactile et/ou plusieurs dates cibles d'inspiration. Les instructions peuvent comprendre également au moins un identifiant d'interface tactile associé à chaque date cible d'interaction tactile. Préférentiellement, les dates cibles sont synchronisées, de manière à suivre un tempo ou une partition d'une musique.

Les indicateurs respiratoires Kp et tactile Ki générés sont alors comparés à la consigne multimédia Cm. Notamment, l'horodatage des indicateurs respiratoires Kp et tactiles Ki sont comparés aux dates cibles comprises dans la consigne multimédia Cm. La donnée respiratoire S peut être générée en fonction des écarts entre les horodatages des indicateurs et les dates cibles de la consigne multimédia. Dans un mode de réalisation, les identifiants des dates cibles tactiles sont comparés aux identifiants des indicateurs tactiles Ki générés et la donnée respiratoire est générée en fonction de cette comparaison.

D'une part, la présence de consignes tactiles permet à l'utilisateur de moins se focaliser sur sa respiration, et donc d'obtenir des résultats plus représentatifs des capacités de l'utilisateur. D'autre part, les consignes tactiles permettent à l'utilisateur de fixer une synchronicité sur laquelle il peut s'appuyer pour synchroniser ses expirations.

Dans un deuxième exemple, la consigne multimédia Cm peut consister à déplacer un élément contrôlable d'un jeu vidéo interactif affiché sur l'afficheur 11. L'élément contrôlable 33 est contrôlable par l'expiration de l'utilisateur et/ou par les interactions sur les interfaces tactiles. Dans un exemple illustré sur la figure 4, l'émission de la consigne multimédia Cm comprend l'affichage d'un élément contrôlable 33 d'un jeu vidéo interactif (ici un bateau). Le bateau est contrôlable par l'expiration de l'utilisateur pour faire avancer le bateau linéairement et par une interaction tactile pour faire sauter le bateau ou inversement. Une barre d'expiration 31 permet à l'utilisateur de visualiser la variation de l'indicateur respiratoire Kp. L'indicateur respiratoire Kp permet de faire avancer l'élément contrôlable 33. La consigne multimédia Cm peut comprendre également l'affichage d'obstacles tels des crottes de mouettes 32 et des rochers 37. La consigne multimédia Cm peut comprendre alors une instruction d'évitement desdits obstacles 32, 37 en avançant et en sautant. La consigne multimédia Cm peut comprendre une instruction d'interaction avec au moins une interface tactile lorsque l'élément contrôlable 33 est à proximité ou au contact d'un obstacle, par exemple pour ramasser des objets.

La donnée respiratoire S est alors générée en fonction du nombre d'obstacles évités et en fonction du temps pour terminer un parcours et/ou en fonction de la distance parcourue par l'élément contrôlable 33 dans le jeu interactif en un temps donné. Un score 34 et un chronomètre 36 peuvent être affichés.

Dans un troisième exemple, la consigne multimédia Cm comprend l'affichage d'un parcours comprenant un départ et une arrivée et d'un élément contrôlable à faire avancer depuis le départ vers l'arrivée. Le déplacement de l'élément contrôlable est activé par l'indicateur respiratoire et la direction de déplacement peut être contrôlée par l'indicateur tactile ou inversement. La donnée respiratoire est alors fonction du temps pris pour terminer le parcours et/ou fonction du nombre d'obstacles évités.

Dans un autre exemple, la consigne multimédia Cm comprend une instruction de respiration comprenant une durée d'expiration cible et/ou une force d'expiration cible à atteindre par l'utilisateur. L'instruction de respiration peut comprendre un nombre d'expirations cibles à atteindre sur une durée prédéfinie. Les instructions d'interaction peuvent comprendre une interaction cible simultanée à une expiration cible. La donnée de respiration S peut alors être générée en fonction de l'indicateur respiratoire Kp. La donnée respiratoire S peut être générée de manière à correspondre à une constante respiratoire telle que la capacité pulmonaire, le volume expiratoire maximal par seconde ou le débit expiratoire de pointe.

Dans un mode de réalisation, le dispositif 100 comprend des moyens de connexion à un réseau. Le dispositif 100 peut être conçu pour comparer la donnée respiratoire S générée avec des données respiratoires d'autres utilisateurs en temps réel ou en utilisant des données d'historique d'utilisation. Dans un mode de réalisation, un dispositif peut alors générer des consignes multimédias et les transmettre via le réseau au dispositif de chaque utilisateur. Ainsi, chaque utilisateur peut recevoir les mêmes consignes multimédias, permettant une utilisation de type « multi-joueurs ».

Dans un mode de réalisation, le dispositif 100 comprend une pluralité d'unités de respiration 1 et un terminal électronique 10. Le terminal électronique est conçu pour être connecté à au moins deux unités de respiration 1 simultanément pour générer simultanément une donnée respiratoire S pour chaque unité de respiration 1 simultanément. Un avantage est de permettre d'utiliser le dispositif en multijoueur avec un ami ou un personnel soignant.

Dans un mode d'exécution de l'invention, la méthode comprend une étape préalable de détection de l'embout buccal amovible 5. Le type d'embout buccal amovible 5 connecté à l'unité de respiration 1 est ainsi déterminé. La consigne multimédia Cm peut être générée en fonction du type d'embout buccal amovible 5 connecté à l'unité de respiration 1 qui a été détecté. Dans un mode alternatif, une interface du dispositif permet à l'utilisateur de sélectionner le type d'embout buccal amovible A, B, C.

Les données respiratoires S peuvent être stockées sur une mémoire du terminal électronique, transmises à un réseau et/ou transmise à un appareil d'un tiers. Un avantage est donc de pouvoir suivre la progression de l'utilisateur en fonction de la période de rééducation ou de traitement. Un autre avantage est de transmettre ou de rendre accessible les données à un proche de l'utilisateur et/ou à un personnel soignant engagé dans le suivi respiratoire de l'utilisateur. Les données respiratoires peuvent comprendre des constantes respiratoires de l'utilisateur. Les données respiratoires S peuvent être présentées sous la forme d'un historique, d'un graphique ou d'un tableau évolutif. La transmission à un réseau ou à un tiers peut être réalisée par l'activation de l'utilisateur, par exemple par l'envoi d'un mail ou d'un transfert direct depuis le terminal électronique 10. Les données respiratoires peuvent être cryptées ou protégées par une clé de sécurisation ou par un mot de passe.

Préférentiellement, le terminal électronique 10 du dispositif 100 comprend un calculateur CALC adapté ou conçu pour exécuter les étapes de la méthode de génération d'une donnée respiratoire selon l'invention.

Selon un autre aspect, l'invention concerne un produit programme d'ordinateur comprenant des instructions qui conduisent le dispositif 100 selon l'invention à exécuter les étapes de la méthode selon l'invention.

Le terminal électronique 10 peut comprendre un support lisible par le calculateur CALC ou par un ordinateur, sur lequel est enregistré ledit programme d'ordinateur. Ledit support peut comprendre une mémoire non transitoire.

Selon un autre aspect, l'invention concerne un tel support sur lequel est enregistré ledit programme d'ordinateur.

En utilisant les interfaces tactiles en plus de la respiration, le dispositif et la méthode selon l'invention autorisent ainsi une plus grande variété d'exercices interactifs, permettent de détourner l'attention de l'utilisateur sur sa respiration et permettent d'assister l'utilisateur à atteindre un rythme d'expiration cible en jouant le même rythme ou un rythme associé avec un ou plusieurs doigts en même temps. Par rythme associé, on entend un rythme partageant avec le rythme d'expiration un même tempo.

Le dispositif permet ainsi de travailler l'expiration de l'utilisateur, à la fois au niveau des capacités pulmonaires (volume d'air expiré, puissance de l'expiration) et à la fois au niveau de la réactivité des muscles (vitesse de réaction, capacité à maintenir un rythme) en fonction des différentes consignes multimédia.

Selon un autre aspect, l'unité de respiration comprend des moyens permettant de déterminer l'orientation de ladite unité de respiration.

L'unité de respiration peut comprendre à ce titre un capteur de mouvement. Le capteur de mouvement peut comprendre un ou plusieurs accéléromètres pour calculer l'accélération linéaire selon 1 axe. Préférentiellement, le capteur de mouvement comprend 3 accéléromètres pour calculer une accélération linéaire selon 3 axes orthogonaux. Dans un mode de réalisation, le capteur de mouvement comprend un ou plusieurs gyromètres pour calculer une rotation ou une vitesse angulaire selon un angle tel que l'angle de roulis, de tangage ou de cap). Préférentiellement, le capteur de mouvement comprend 3 gyromètres pour calculer une rotation ou une vitesse angulaire selon 3 axes orthogonaux.

Dans un mode de réalisation, le capteur de mouvement comprend une centrale à inertie. La centrale à inertie comprend préférentiellement 3 accéléromètres pour calculer une accélération linéaire selon 3 axes orthogonaux et 3 gyromètres pour calculer une accélération angulaire selon 3 axes orthogonaux. Le capteur de mouvement est préférentiellement intégré à l'unité de respiration du dispositif.

Le capteur de mouvement est connecté au terminal électronique de manière à communiquer les données mesurées au terminal électronique.

Le dispositif est conçu pour générer l'orientation de l'unité de respiration. Le dispositif est préférentiellement configuré pour générer et afficher un indicateur d'orientation. L'indicateur d'orientation peut être calculé en fonction de l'orientation de l'unité de respiration et/ou en fonction d'une orientation cible prédéterminée. L'affichage d'un tel indicateur peut être affiché sur un afficheur ou peut être généré par les diodes du dispositif. Par orientation, on peut entendre préférentiellement l'angle d'inclinaison de l'unité de respiration par rapport à un plan horizontal du référentiel terrestre.

L'avantage d'un tel mode est décrit ci-dessous.

L'unité de respiration peut être utilisée dans le cadre de l'assistance à la prise d'un agent thérapeutique pour des maladies respiratoires. Cet agent peut comprendre une poudre médicamenteuse, un gaz médicamenteux ou une solution nébulisée médicamenteuse. L'agent doit être aspiré par l'utilisateur selon une orientation particulière.

Dans un mode de réalisation, le dispositif est configuré pour envoyer à l'utilisateur un message lorsque l'orientation de l'unité de respiration est comprise dans une plage angulaire cible prédéterminée.

Dans certains cas, l'agent doit être inspiré par l'utilisateur selon une orientation particulière. Dans un mode de réalisation, le dispositif est configuré pour transmettre à l'utilisateur un message lorsque la force d'inspiration mesurée par l'unité de respiration est comprise dans une plage cible prédéterminée.

Le message peut comprendre une alerte sonore et/ou un changement de couleur d'une diode et/ou la génération d'une vibration de l'unité de respiration et/ou un message affiché sur un afficheur.

L'utilisateur est alors avantageusement assisté pour réaliser une inspiration de l'agent médicamenteux avec l'orientation et/ou la force d'inspiration adéquate.

Dans un mode de réalisation, l'embout peut comprendre un réceptacle pour la réception ou le stockage d'un tel agent thérapeutique de telle manière à être inspirée par l'utilisateur inspirant par l'ouverture de l'embout.

Dans un autre exemple, l'unité de respiration est utilisée pour reproduire la prise d'un tel agent thérapeutique. L'unité de respiration permet dans ce cas de fournir à l'utilisateur un moyen d'entrainement pour simuler la prise d'un tel agent thérapeutique, notamment pour s'entrainer sur l'orientation du dispositif et la force d'inspiration à exercer pour prendre convenablement l'agent thérapeutique.

## Revendications

1. Dispositif (100) de génération d'une donnée respiratoire d'un utilisateur comprenant :
▪ une unité de respiration (1) comprenant :
▪ un capteur de pression d'air (82) pour mesurer la pression d'air expiré et/ou inspiré par l'utilisateur dans une chambre fluidique d'expiration (8) ;
▪ au moins deux interfaces tactiles (2) ;
▪ un terminal électronique (10) comprenant un calculateur (CALC) adapté pour exécuter :
▪ une génération (CO) d'une consigne multimédia (Cm) horodatée et la transmission de cette consigne multimédia (Cm) à l'utilisateur par un moyen de transmission (11) ;
▪ une mesure (MES_P) d'une pression d'air par le capteur de pression d'air (82) ;
▪ une génération (GEN_P) d'un indicateur respiratoire horodaté (Kp) en fonction de la pression d'air mesurée,
▪ une détection (DET) d'une interaction (SI) sur au moins une des interfaces tactiles (2) ;
▪ une génération (GEN_I) d'un indicateur tactile horodaté (Ki) en fonction de l'interaction détectée (SI) ;
▪ une réception de l'indicateur respiratoire (Kp) et de l'indicateur tactile (Ki) ;
▪ une génération (DAT) d'une donnée respiratoire (S) quantifiant une performance respiratoire de l'utilisateur en fonction de la corrélation de l'indicateur tactile (Ki) et de l'indicateur respiratoire (Kp) avec la consigne multimédia (Cm).

2. Dispositif selon la revendication 1, comprenant en outre un oxymètre à réflexion (3) connecté au terminal électronique.

3. Dispositif selon la revendication 1 ou la revendication 2 comprenant en outre un afficheur (11) pour afficher la consigne multimédia (Cm).

4. Dispositif selon l'une des revendications 1 à 3, dans lequel la consigne multimédia horodatée comprend une instruction de respiration et une instruction d'interaction.

5. Dispositif selon la revendication 4, dans lequel l'instruction d'interaction comprend une date de départ et dans laquelle la donnée respiratoire (S) est générée en fonction de ladite date de départ et de l'horodatage de l'indicateur tactile.

6. Dispositif selon l'une des revendications 1 à 5, l'unité de respiration comprenant en outre un moyen de mesure (3) d'une valeur physiologique du sujet, et dans lequel le calculateur est adapté pour exécuter en outre une mesure préalable physiologique de l'utilisateur par le moyen de mesure (3) et pour générer une alerte si ladite mesure est en-dehors d'une plage de valeurs prédéfinie.

7. Dispositif selon l'une des revendications précédentes, dans lequel l'unité de respiration comprend en outre au moins un capteur de mouvement pour mesurer un angle d'inclinaison de ladite unité de respiration.

8. Dispositif selon la revendication 7, dans lequel la mesure physiologique comprend une fréquence cardiaque et/ou un taux de saturation en oxygène dans le sang mesurés par un oxymètre à réflexion arrangé sur la surface de l'unité de respiration.

9. Dispositif selon l'une des revendications 1 à 8, comprenant en outre un afficheur (11) pour l'affichage d'une image d'un jeu vidéo interactif sur un le jeu vidéo interactif incluant un élément contrôlable (33) en fonction de l'indicateur respiratoire (Kp) et de l'indicateur tactile (Ki) ou en fonction de la donnée respiratoire (S).

10. Produit programme d'ordinateur comprenant des instructions qui, lorsque le programme est exécuté par un ordinateur, conduisent le dispositif selon l'une des revendications 1 à 9 à mettre en oeuvre la méthode suivante :
▪ une génération (CO) d'une consigne multimédia (Cm) horodatée par un logiciel d'un terminal électronique (10) et la transmission de cette consigne multimédia (Cm) à l'utilisateur par un moyen de transmission (11) ;
▪ une mesure (MES_P) d'une pression d'air dans une chambre fluidique d'expiration (8) d'une unité de respiration (1) pour recevoir un volume d'air expiré et/ou inspiré d'un utilisateur ;
▪ une génération (GEN_P) d'un indicateur respiratoire horodaté (Kp) en fonction de la pression d'air mesurée,
▪ une détection (DET) d'une interaction (SI) sur une interface tactile (2);
▪ une génération (GEN_I) d'un indicateur tactile horodaté (Ki) en fonction de l'interaction détectée (SI) ;
▪ une réception par un calculateur (CALC) du terminal électronique (10) de l'indicateur respiratoire (Kp) et de l'indicateur tactile (Ki) ;
▪ une génération (DAT) d'une donnée respiratoire (S) quantifiant une performance respiratoire de l'utilisateur en fonction de la corrélation de l'indicateur tactile (Ki) et de l'indicateur respiratoire (Kp) avec la consigne multimédia (Cm).

11. Produit programme d'ordinateur selon la revendication 10, dans lequel la consigne multimédia horodatée comprend une instruction de respiration et une instruction d'interaction.

12. Produit programme d'ordinateur selon la revendication 11, dans laquelle l'instruction d'interaction comprend une date de départ et dans laquelle la donnée respiratoire (S) est générée en fonction de ladite date de départ et de l'horodatage de l'indicateur tactile.

13. Produit programme d'ordinateur selon l'une des revendications 10 à 12, dans lequel la méthode comprend en outre une mesure préalable physiologique de l'utilisateur et l'émission d'une alerte si la mesure est dehors d'une plage de valeurs prédéfinie.

14. Produit programme d'ordinateur selon la revendication 13, dans laquelle la mesure physiologique comprend une fréquence cardiaque et/ou un taux de saturation en oxygène dans le sang mesurés par un oxymètre à réflexion arrangé sur la surface de l'unité de respiration.

15. Produit programme d'ordinateur selon l'une des revendications 10 à 14, dans lequel la méthode mise en oeuvre comprend en outre l'affichage d'une image d'un jeu vidéo interactif sur un afficheur (11), le jeu vidéo interactif incluant un élément contrôlable (33) en fonction de l'indicateur respiratoire (Kp) et de l'indicateur tactile (Ki) ou en fonction de la donnée respiratoire (S).

## Patentansprüche

1. Vorrichtung (100) zur Erzeugung eines Atemdatenwerts eines Benutzers, umfassend:
▪ eine Atemeinheit (1), umfassend:
▪ einen Luftdrucksensor (82) zur Messung des Drucks der vom Benutzer ausgeatmeten und/oder eingeatmeten Luft in einer fluidischen Expirationskammer (8);
▪ mindestens zwei berührungsempfindliche Schnittstellen (2);
▪ ein elektronisches Endgerät (10), das einen Rechner (CALC) umfasst, der geeignet ist, auszuführen:
▪ eine Erzeugung (CO) eines zeitgestempelten Multimedia-Sollwerts (Cm) und die Übertragung dieses Multimedia-Sollwerts (Cm) an den Benutzer über ein Übertragungsmittel (11);
▪ eine Messung (MES_P) eines Luftdrucks durch den Luftdrucksensor (82);
▪ eine Erzeugung (GEN_P) eines zeitgestempelten Atemindikators (Kp) in Abhängigkeit von dem gemessenen Luftdruck,
▪ eine Ermittlung (DET) einer Interaktion (SI) auf mindestens einer der berührungsempfindlichen Schnittstellen (2);
▪ eine Erzeugung (GEN_I) eines zeitgestempelten Berührungsindikators (Ki) in Abhängigkeit von der ermittelten Interaktion (SI);
▪ einen Empfang des Atemindikators (Kp) und des Berührungsindikators (Ki);
▪ eine Erzeugung (DAT) eines Atemdatenwerts (S), der eine Atemleistung des Benutzers in Abhängigkeit von der Korrelation des Berührungsindikators (Ki) und des Atemindikators (Kp) mit dem Multimedia-Sollwert (Cm) quantifiziert.

2. Vorrichtung nach Anspruch 1, die ferner ein Reflexionsoximeter (3) umfasst, das mit dem elektronischen Endgerät verbunden ist.

3. Vorrichtung nach Anspruch 1 oder Anspruch 2, die ferner ein Display (11) zur Anzeige des Multimedia-Sollwerts (Cm) umfasst.

4. Vorrichtung nach einem der Ansprüche 1 bis 3, wobei der zeitgestempelte Multimedia-Sollwert eine Atemanweisung und eine Interaktionsanweisung umfasst.

5. Vorrichtung nach Anspruch 4, wobei die Interaktionsanweisung ein Startdatum umfasst und wobei der Atemdatenwert (S) in Abhängigkeit von dem Startdatum und der Zeitstempelung des Berührungsindikators erzeugt wird.

6. Vorrichtung nach einem der Ansprüche 1 bis 5, wobei die Atemeinheit ferner ein Messmittel (3) eines physiologischen Wertes des Probanden umfasst, und wobei der Rechner dazu geeignet ist, ferner eine vorherige physiologische Messung des Benutzers durch das Messmittel (3) durchzuführen und eine Warnung zu erzeugen, wenn diese Messung außerhalb eines vordefinierten Wertebereichs liegt.

7. Vorrichtung nach einem der vorhergehenden Ansprüche, wobei die Atemeinheit ferner wenigstens einen Bewegungssensor zum Messen eines Neigungswinkels der Atemeinheit umfasst.

8. Vorrichtung nach Anspruch 7, wobei die physiologische Messung eine Herzfrequenz und/oder eine Sauerstoffsättigungsrate im Blut umfasst, die von einem Reflexionsoximeter gemessen werden, das auf der Oberfläche der Atemeinheit angeordnet ist.

9. Vorrichtung nach einem der Ansprüche 1 bis 8, die ferner ein Display (11) zur Anzeige eines Bildes eines interaktiven Videospiels auf einem interaktiven Videospiel umfasst, das ein steuerbares Element (33) in Abhängigkeit vom Atemindikator (Kp) und vom taktilen Indikator (Ki) oder in Abhängigkeit von dem Atemdatenwert (S) einschließt.

10. Computerprogrammprodukt, das Anweisungen umfasst, die, wenn das Programm von einem Computer ausgeführt wird, die Vorrichtung nach einem der Ansprüche 1 bis 9 dazu veranlassen, das folgende Verfahren umzusetzen:
▪ eine Erzeugung (CO) eines zeitgestempelten Multimedia-Sollwerts (Cm) durch eine Software eines elektronischen Endgeräts (10) und die Übertragung dieses Multimedia-Sollwerts (Cm) an den Benutzer über ein Übertragungsmittel (11);
▪ eine Messung (MES_P) eines Luftdrucks in einer fluidischen Expirationskammer (8) einer Atemeinheit (1), um ein Volumen an ausgeatmeter und/oder eingeatmeter Luft eines Benutzers aufzunehmen;
▪ eine Erzeugung (GEN_P) eines zeitgestempelten Atemindikators (Kp) in Abhängigkeit von dem gemessenen Luftdruck,
▪ eine Ermittlung (DET) einer Interaktion (SI) auf einer berührungsempfindlichen Schnittstelle (2);
▪ eine Erzeugung (GEN_I) eines zeitgestempelten Berührungsindikators (Ki) in Abhängigkeit von der ermittelten Interaktion (SI);
▪ einen Empfang durch einen Rechner (CALC) des elektronischen Endgeräts (10) des Atemindikators (Kp) und des Berührungsindikators (Ki);
▪ eine Erzeugung (DAT) eines Atemdatenwerts (S), der eine Atemleistung des Benutzers in Abhängigkeit von der Korrelation des Berührungsindikators (Ki) und des Atemindikators (Kp) mit dem Multimedia-Sollwert (Cm) quantifiziert.

11. Computerprogrammprodukt nach Anspruch 10, wobei der zeitgestempelte Multimedia-Sollwert eine Atemanweisung und eine Interaktionsanweisung umfasst.

12. Computerprogrammprodukt nach Anspruch 11, wobei die Interaktionsanweisung ein Startdatum umfasst und wobei der Atemdatenwert (S) in Abhängigkeit von dem Startdatum und der Zeitstempelung des Berührungsindikators erzeugt wird.

13. Computerprogrammprodukt nach einem der Ansprüche 10 bis 12, wobei das Verfahren ferner eine vorherige physiologische Messung des Benutzers und die Ausgabe einer Warnung umfasst, wenn die Messung außerhalb eines vordefinierten Wertebereichs liegt.

14. Computerprogrammprodukt nach Anspruch 13, wobei die physiologische Messung eine Herzfrequenz und/oder eine Sauerstoffsättigungsrate im Blut umfasst, die von einem Reflexionsoximeter gemessen werden, das auf der Oberfläche der Atemeinheit angeordnet ist.

15. Computerprogrammprodukt nach einem der Ansprüche 10 bis 14, wobei das durchgeführte Verfahren ferner die Anzeige eines Bildes eines interaktiven Videospiels auf einem Display (11) umfasst, wobei das interaktive Videospiel ein steuerbares Element (33) in Abhängigkeit von dem Atemindikator (Kp) und dem Berührungsindikator (Ki) oder in Abhängigkeit von dem Atemdatenwert (S) einschließt.

## Claims

1. Device (100) for generating a respiratory datum for a user comprising:
▪ a respiration unit (1) comprising:
▪ an air pressure sensor (82) for measuring the air pressure exhaled and/or inhaled by the user in a fluid exhalation chamber (8);
▪ at least two tactile interfaces (2);
▪ an electronic terminal (10) comprising a calculator (CALC) adapted to execute:
▪ generating (CO) a multimedia instruction (Cm) that is timestamped and transmitting this multimedia instruction (Cm) to the user via a transmission means (11);
▪ measuring (MES_P) an air pressure by the air pressure sensor (82);
▪ generating (GEN_P) a timestamped respiratory indicator (Kp) as a function of the measured air pressure,
▪ detecting (DET) an interaction (SI) on at least one of the tactile interfaces (2)
▪ generating (GEN_I) a timestamped tactile indicator (Ki) as a function of the interaction detected (SI);
▪ reception of the respiratory indicator (Kp) and the tactile indicator (Ki);
▪ generating (DAT) a respiratory datum (S) quantifying the user's respiratory performance as a function of the correlation of the tactile indicator (Ki) and the respiratory indicator (Kp) with the multimedia instruction (Cm).

2. Device according to claim 1, further comprising a reflection oximeter (3) connected to the electronic terminal.

3. Device according to claim 1 or claim 2 further comprising a display (11) for displaying the multimedia instruction (Cm).

4. Device according to one of claims 1 to 3, wherein the timestamped multimedia instruction comprises a respiration instruction and an interaction instruction.

5. Device according to claim 4, wherein the interaction instruction comprises a start date and wherein the respiratory datum (S) is generated as a function of said start date and the timestamping of the tactile indicator.

6. Device according to one of claims 1 to 5, wherein the respiratory unit further comprises a means of measuring (3) a physiological value of the subject, and wherein the calculator is adapted to further perform a prior physiological measurement of the user by the measuring means (3) and to generate an alert if said measurement is outside a predefined range of values.

7. Device according to one of the preceding claims, wherein the respiration unit further comprises at least one motion sensor to measure an inclination angle of said respiration unit.

8. Device according to claim 7, wherein the physiological measurement comprises a heart rate and/or blood oxygen saturation rate measured by a reflection oximeter arranged on the surface of the respiration unit.

9. Device according to one of claims 1 to 8, further comprising a display (11) for displaying an image of an interactive video game on an interactive video game including a controllable element (33) as a function of the respiratory indicator (Kp) and the tactile indicator (Ki) or as a function of the respiratory datum (S).

10. Computer program product comprising instructions which, when the program is run by a computer, lead the device according to one of the claims 1 to 9 to implement the following method:
▪ generating (OC) a multimedia instruction (Cm) that is timestamped via software of an electronic terminal (10) and transmitting this multimedia instruction (Cm) to the user via a transmission means (11);
▪ measuring (MES_P) an air pressure in a fluid exhalation chamber (8) of a respiration unit (1) to receive a volume of air exhaled and/or inhaled by a user;
▪ generating (GEN_P) a timestamped respiratory indicator (Kp) as a function of the measured air pressure;
▪ detecting (DET) an interaction (SI) on a tactile interface (2);
▪ generating (GEN_I) a timestamped tactile indicator (Ki) as a function of the interaction detected (SI);
▪ reception by a calculator (CALC) of the electronic terminal (10) of the respiratory indicator (Kp) and the tactile indicator (Ki);
▪ generating (DAT) a respiratory datum (S) quantifying the user's respiratory performance as a function of the correlation of the tactile indicator (Ki) and the respiratory indicator (Kp) with the multimedia instruction (Cm).

11. Computer program product according to claim 10, wherein the timestamped multimedia instruction comprises a respiration instruction and an interaction instruction.

12. Computer program product according to claim 11, wherein the interaction instruction comprises a start date and wherein the respiratory datum (S) is generated according to said start date and timestamping of the tactile indicator.

13. Computer program product according to one of claims 10 to 12, wherein the method comprises a prior physiological measurement of the user and the issuance of an alert if the measurement is outside a predefined range of values.

14. Computer program product according to claim 13, wherein the physiological measurement comprises a heart rate and/or blood oxygen saturation rate measured by a reflection oximeter arranged on the surface of the respiration unit.

15. Computer program product according to one of claims 10 to 14, wherein the method comprises the display of an image of an interactive video game on a display (11), the interactive video game including a controllable element (33) as a function of the respiratory indicator (Kp) and the tactile indicator (Ki) or as a function of the respiratory datum (S).
